# EUROPEAN PATENT SPECIFICATION

(11) **EP 4 436 552 B1**
(45) Date of publication and mention of the grant of the patent: **04.02.2026**
(21) Application number: 22809767.1
(22) Date of filing: 26.10.2022
(51) Int. Cl.: A61K 9/20, A61K 31/167, A61P 23/02, A61K 9/00

(54) **GASTRO-RETENTIVE ORAL DOSAGE UNIT CONTAINING A LOCAL ANAESTHETIC**
GASTRORETENTIVE ORALE DOSIEREINHEIT MIT LOKALANÄSTHETIKUM
UNITÉ POSOLOGIQUE ORALE GASTRO-RÉTENTIVE CONTENANT UN ANESTHÉSIQUE LOCAL

(30) Priority: 25.11.2021 EP 21210588
(43) Date of publication of application: 02.10.2024
(73) Proprietor: Orexa B.V., 5373 KH Herpen (NL)
(72) Inventor: VAN DEN HEUVEL, Dennie, 5373 KH Herpen (NL); PEETERS, Bernardus Wijnand Mathijs Marie, 5373 KH Herpen (NL)
(74) Representative: Nederlandsch Octrooibureau
(86) International application number: PCT/EP2022/079975
(87) International publication number: WO 2023/094100

(56) References cited:
- WO-A1-2013/171252
- US-A- 5 254 591
- US-A1- 2011 287 096
- US-B2- 7 776 345
- BARDONNET P L ET AL: "Gastroretentive dosage forms: Overview and special case of Helicobacter pylori", JOURNAL OF CONTROLLED RELEASE, ELSEVIER, AMSTERDAM, NL, vol. 111, no. 1-2, 10 March 2006 (2006-03-10), pages 1 - 18, XP024957424, ISSN: 0168-3659, [retrieved on 20060310], DOI: 10.1016/J.JCONREL.2005.10.031

## Description

### TECHNICAL FIELD OF THE INVENTION

The present invention relates to a gastro-retentive oral dosage unit for use in the treatment or prevention of malnutrition in subjects having adequate access to nutrition, or in the treatment or prevention of early or excessive satiety, containing a local anaesthetic.

### BACKGROUND OF THE INVENTION

'Anorexia of aging' refers to reduced appetite and energy intakes observed in some older adults. Satiation (the process that leads to the termination of eating, which may be accompanied by a feeling of satisfaction) and satiety (the feeling of fullness that persists after eating, potentially suppressing further energy intake until hunger returns) are important factors in the control of appetite and energy intake, and there is evidence that some aspects of satiation and satiety are altered in older adults.

Cachexia is a complex syndrome associated with an underlying illness causing ongoing muscle loss that is insufficiently reversed with nutritional supplementation. A range of diseases can cause cachexia, most commonly cancer, congestive heart failure, chronic obstructive pulmonary disease, chronic kidney disease and AIDS. Early (premature) satiety has been identified as a contributing factor. In contrast to weight loss from inadequate caloric intake, cachexia causes mostly muscle loss instead of fat loss.

Next to chronic conditions of early or excessive satiety, also acute conditions exist. After abdominal surgery many patients have problems starting eating. Lack of appetite or excessive satiety can result in post-operative ileus. Post-operative ileus is characterized by the inability to tolerate a solid diet, delayed passage of flatus and formed stool, pain and abdominal distension, nausea, vomiting, and accumulation of gas or fluids in the bowel. Post-operative ileus results frequently in a prolonged hospital stay.

Local anaesthetics such as lidocaine, create an absence of pain in a specific location of the body without a loss of consciousness, as opposed to a general anesthetic. Local anaesthetics belong to one of two classes: aminoamide and aminoester local anesthetics. Synthetic local anaesthetics are structurally related to cocaine. They differ from cocaine mainly in that they have a very low abuse potential and do not produce hypertension or (with few exceptions) vasoconstriction.

Lidocaine is a local anaesthetic of the amino amide type that is mostly administered either by injection and/or topical/transdermal route. The efficacy profile of lidocaine as a local anaesthetic is characterized by a rapid onset of action and intermediate duration of efficacy.

Lidocaine is one of the most commonly used local anaesthetics in dentistry. For surface anaesthesia, several formulations can be used for endoscopies, before intubations, etc. Buffering the pH of lidocaine makes local numbing less painful. Lidocaine drops can be used on the eyes for short ophthalmic procedures.

Trachisan^{®} is a commercially available lidocaine lozenge that provides relief of a sore throat.

Quadeib et al. (The Oral Administration of Lidocaine HCI Biodegradable Microspheres: Formulation and Optimization, Int J Nanomedicine. 2020; 15: 857-869.) describe an oral lidocaine formulation that improves the solubility of lidocaine and enhances the release of lidocaine *in vitro.* Different ratios of alginate and chitosan were used to create different types of microspheres. According to the authors the microspheres can be used as a novel oral delivery system for lidocaine, exhibiting significantly less cardiac toxicity than lidocaine alone.

WO 2013/171252 describes a method to facilitate food intake and food retention in a mammal by applying a sufficient dose of a local anesthetic drug in the lumen of the pharyngeal-esophageal-gastric-duodenal tract before or during or after eating. Lidocaine is mentioned as an example of a local anaesthetic drug.

US 2011/0287096 describes an oral dosage form comprising,
(a) a gastroretentive component comprising a basic amine drug, an acidifier, a carbonic compound, a hydrophilic insoluble polymer, and a water insoluble fluid penetrating agent; and
(b) a non-gastroretentive component comprising a basic amine drug, an acidifier and a sustained release polymer.

US 2020/0316150 describes a sustained release gastroretentive swellable oral formulation comprising: bergenin-rich *Bergenia ciliata* hydroalcoholic extract or fraction; gastroretentive expandable polymers selected from the group consisting of hydroxypropylmethylcellulose (HPMC) K15M, HPMC K30M, HPMC K100M, xanthan gum, chitosan, ethyl cellulose of viscosity in the range of 10-100 cps, sodium alginate, and combination thereof; and optionally an excipient selected from the group consisting of a carrier, an adjuvant, and a binder.

### SUMMARY OF THE INVENTION

The inventors have developed a dosage unit that is particularly suited for treating or preventing malnutrition in subjects having adequate access to nutrition, or for treating or preventing early or excessive satiety. The dosage unit according to the present invention can be taken orally after which it gradually releases local anaesthetic in the stomach over a prolonged period of time.

The gastro-retentive oral dosage unit of the present invention is for use in the treatment or prevention of malnutrition in subjects having adequate access to nutrition, or in the treatment or prevention of early or excessive satiety, said use comprising oral administration of a dosage unit having a weight of 150-1,000 mg and comprising 70-100 wt.% of a core unit and 0-30 wt.% of a coating, the core unit comprising:
(a) 15-50 wt.% local anaesthetic;
(b) 2-20 wt.% of effervescent;
(c) 8-30 wt.% of hydrophilic polymer;
(d) 5-75 wt.% of excipient.

Although the inventors do not wish to be bound by theory, it is believed that when the dosage unit of the present invention comes into contact with gastric fluid, the effervescent starts releasing gas bubbles whilst at the same time the hydrophilic polymer starts forming a moist matrix that captures these gas bubbles. The buoyancy resulting from this joint action causes the dosage unit to float, thereby increasing the residence time of the dosage unit within the stomach. Since the moist polymer matrix prevents or at least delays disintegration of the dosage unit, the local anaesthetic is gradually released. Gradual and prolonged release of the local anaesthetic is important to achieve a sustained effect, especially if the local anaesthetic is inactivated when it comes into contact with gastric fluid.

The gastro-retentive oral dosage unit of the present invention offers the advantage that it is easy to manufacture as it does not, for instance, comprise an elastic membrane. Furthermore, the release characteristics of the dosage unit can effectively and easily be manipulated by adjusting the concentration levels of the components of the core unit. After oral administration of the dosage unit local anaesthetic is gradually released in the stomach during a prolonged period of time, causing suppression of early satiety feelings. This suppression of satiety feelings is believed to be caused by the relaxation of muscle fibres in the stomach wall tissue that is induced by the local anaesthetic.

Also provided (not claimed) is a process of preparing the dosage unit of the present invention, said process comprising:
- preparing a tableting mixture by intimately mixing components (a), (b), (c) and (d): and
- compressing the tableting mixture into tablets.

### DETAILED DESCRIPTION OF THE INVENTION

Accordingly, a first aspect of the invention relates to a gastro-retentive oral dosage unit for use in the treatment or prevention of malnutrition in subjects having adequate access to nutrition, or in the treatment or prevention of early or excessive satiety, said use comprising oral administration of a dosage unit having a weight of 150-1,000 mg, said dosage unit comprising 70-100 wt.% of a core unit and 0-30 wt.% of a coating, the core unit comprising:
(a) 15-50 wt.% local anaesthetic;
(b) 2-20 wt.% of effervescent;
(c) 8-30 wt.% of hydrophilic polymer;
(d) 5-75 wt.% of excipient.

The term "local anaesthetic" as used herein refers to a medication that creates an absence of pain in a specific location of the body without a loss of consciousness. These local anaesthetics may be present in the dosage unit in any pharmaceutically acceptable form, including pharmaceutically acceptable salts.

The term "topical anaesthetic" as used herein refers to a local anaesthetic that creates absence of pain in tissue when topically applied thereon.

The term "lidocaine" as used herein encompasses lidocaine as well as pharmaceutically acceptable salts thereof (such as lidocaine.HCl).

The term "hydrophilic polymer" as used herein refers to a polymer that is water-swellable and/or water-soluble.

The dosage unit of the present invention is preferably provided in the form of a tablet, more preferably of a compressed tablet. The tablet preferably has a tablet strength of at least 40 N, more preferably of 80 to 140 N.

The dosage unit of the present invention offers the advantage that it can be prepared in a very simple manner by intimately mixing ingredients (a) to (d), followed by a tableting and optionally the application of a protective coating. Accordingly, in a preferred embodiment, the components (a) to (d) are intimately mixed.

According to a preferred embodiment, the local anaesthetic that is employed in accordance with the present invention is a topical aneasthetic. Topical anaesthetics offer the advantage that they are very effective in low dosages.

The local anaesthetic is preferably selected from lidocaine, benzocaine, bupivacaine, oxethazaine, prilocaine, butanilicaine, carticaine, cinchocaine, clibucaine, etidocaine, mepivacaine, ropivacaine, tolycaine, trimecaine, vadocaine, articaine, levobupivacaine, amylocaine, cocaine, propanocaine, clormecaine, cyclomethycaine, proxymetacaine, amethocaine, butacaine, butoxycaine, butyl aminobenzoate, chloroprocaine, dimethocaine, oxybuprocaine, piperocaine, parethoxycaine, procaine, propoxycaine, tricaine and combinations thereof. More preferably, the local anaesthetic is selected from lidocaine, benzocaine, bupivacaine, oxethazaine, prilocaine and combinations thereof. Most preferably, the local anaesthetic is lidocaine.

The local anaesthetic that is employed in accordance with the present invention preferably is an amino amide type local anaesthetic.

The dosage unit of the present invention preferably contains 30-400 mg, more preferably 40-300 mg and most preferably 50-200 mg of the local anaesthetic.

The amount of lidocaine that is contained in the dosage unit is preferably in the range of 30-400 mg, more preferably in the range of 40-300 mg and most preferably in the range of 50-200 mg.

The core unit of the dosage unit of the present invention preferably contains 20-45 wt.%, more preferably 30-40 wt.% of the local anaesthetic.

The core unit of the dosage unit preferably contains 20-45 wt.% lidocaine, more preferably 30-40 wt.%.

Lidocaine may be present in the core unit in any pharmaceutically acceptable form. Preferably, the lidocaine employed is lidocaine hydrochloride salt.

The effervescent in the core unit preferably releases carbon dioxide when it comes into contact with gastric fluid.

According to a particularly preferred embodiment, the effervescent is selected from bicarbonate salts, carbonate salts and combinations thereof. More preferably, the effervescent is selected from ammonium bicarbonate, calcium bicarbonate, lithium bicarbonate, magnesium bicarbonate, potassium bicarbonate, sodium bicarbonate, arginine carbonate, ammonium carbonate, calcium carbonate, lysine carbonate, potassium magnesium carbonate, sodium carbonate, sodium glycine carbonate, sodium sesquicarbonate, zinc carbonate and combinations thereof. Even more preferably, the effervescent is selected from sodium bicarbonate, potassium bicarbonate, sodium carbonate, potassium carbonate and combinations thereof. Most preferably, the effervescent is sodium bicarbonate.

The core unit of the dosage unit preferably comprises 3-18 wt.%, more preferably 4-16 wt.% and most preferably 4-12 wt.% of effervescent.

The hydrophilic polymer that is present in the core unit is preferably selected from hydroxypropyl methylcellulose, hydroxypropyl cellulose, hydroxyethyl cellulose, ethylcellulose, methylcellulose, carboxymethyl cellulose, carboxymethyl starch, polyethylene oxide, carbomer, polyvinyl alcohol, polyvinylpyrrolidone, vinylpyrrolidone-vinyl acetate copolymer, methacrylic acid copolymers, polyacrylic acids, carrageenan, natural gum, guar gum, tragacanth, acacia gum, locust bean gum, xanthan gum and combinations thereof. More preferably, the hydrophilic polymer is selected from hydroxypropyl methylcellulose, hydroxypropyl cellulose, hydroxyethyl cellulose, ethylcellulose, methylcellulose, carboxymethyl cellulose and combinations thereof. Most preferably, the hydrophilic polymer is hydroxypropyl methylcellulose (HPMC).

The core unit preferably contains 10-25 wt.%, more preferably 12-20 wt.% of the hydrophilic polymer.

Excipient is preferably contained in the core unit in a concentration of 10-60 wt.%, more preferably of 25-50 wt.% and most preferably of 32-45 wt.%.

According to a particularly preferred embodiment, the combination of components (a) to (d) constitutes at least 75 wt.% of the core unit, more preferably at least 85 wt.% of the core unit and most preferably at least 90 wt.% of the core unit.

The excipient that is contained in the core unit preferably includes a filler selected from lactose, microcrystalline cellulose, mannitol, starch and combinations thereof. More preferably said filler is selected from lactose, microcrystalline cellulose and combinations thereof. Most preferably, the filler comprises microcrystalline cellulose.

**The** core unit preferably comprises 0-40 wt.% of lactose and 0-45 wt.% of microcrystalline cellulose. More preferably, the core unit comprises 1-20 wt.% of lactose and 20-40 wt.% of microcrystalline cellulose. Most preferably, the core unit comprises 2-10 wt.% of lactose and 26-38 wt.% of microcrystalline cellulose.

The core unit preferably contains 20-70 wt.% of filler, more preferably 25-50 wt.% of filler and most preferably 28-45 wt.% of filler.

According to a particularly preferred embodiment, the core unit contains a disintegrant selected from croscarmellose, sodium starch glycolate, crosspovidone and combinations thereof. More preferably, the disintegrant selected from croscarmellose, sodium starch glycolate and combinations thereof.

The core unit preferably contains 0.5-15 wt.% of disintegrant, more preferably 0.7-10 wt.% of disintegrant, even more preferably 0.8-6 wt.% of disintegrant, yet more preferably 1-5 wt.% of disintegrant and most preferably 1.4-4 wt.% of disintegrant.

According to a further preferred embodiment, the core unit contains antacid. Examples of antacids that may be employed include calcium gluconate, calcium citrate, sodium gluconate, sodium citrate, sodium hydroxide, potassium gluconate, potassium citrate, potassium hydroxide, magnesium carbonate, magnesium gluconate, magnesium citrate, magnesium hydroxide, magnesium oxide, aluminum gluconate, aluminum citrate, aluminum hydroxide and combinations thereof.

The core unit preferably contains 5-30 wt.% of antacid, more preferably 8-25 wt.% of antacid.

In a preferred embodiment of the dosage unit, the core unit contains alginate in combination with a water-soluble divalent metal salt, more particularly a water-soluble salt of Ca²⁺ and/or a water-soluble salt of Mg²⁺. The water-soluble divalent metal salt can be a water-soluble antacid. Here "water-soluble" refers to salt that have a solubility of at least 50 mg/L in distilled water that has been acidified to a pH of 1.2 using HCl. When the dosage unit containing alginate and the water-soluble divalent metal salt comes into contact with gastric juice, the divalent metal salt dissolves, thereby releasing the divalent metal, which subsequently crosslinks the alginate under the formation of a gel network.

The water-soluble divalent metal salt preferably is a water-soluble calcium salt. Examples of water-soluble calcium salts that may suitably be employed in the core unit include calcium carbonate, calcium lactate, calcium chloride, calcium phosphate and combinations thereof. Most preferably, the water-soluble divalent metal salt is calcium carbonate.

The alginate employed in the core unit is preferably selected from alginic acid, sodium alginate, potassium alginate and combinations thereof. Most preferably, the alginate is sodium alginate.

Alginate is preferably contained in the core unit in a concentration of 0.01-1 wt.%, more preferably of 0.03-0.5 wt.%.

The water-soluble divalent metal salt is preferably contained in the core unit in a concentration of 1-100 mmol/L, more preferably of 2-20 mmol/L.

According to a particularly preferred embodiment, the core unit comprises a combination of antacid, alginate and water-soluble divalent metal salt.

According to yet another embodiment, the core unit contains lubricant. Examples of lubricants that may be employed include sodium stearate, magnesium stearate, zinc stearate, aluminum stearate, stearic acid, palmitic acid, calcium hydroxide, talc, corn starch, sodium stearyl fumarate, leucine, polyethylene glycol, glyceryl behenate, colloidal silicon dioxide, hydrogenated vegetable oil, mineral oil, waxes and combinations thereof. More preferably, the lubricant is selected from sodium stearate, magnesium stearate, zinc stearate, aluminum stearate and combinations thereof.

The core unit preferably contains 0.2-3 wt.% of lubricant, more preferably 0.3-2 wt.% of lubricant and most preferably 0.5-1.5 wt.% of lubricant.

In order to prevent in-mouth release of the local anaesthetic, the core unit is preferably surrounded by a saliva-resistant coating. The saliva-resistant coating minimizes the undesired release of local anaesthetic in mouth or throat.

The saliva-resistant coating preferably comprises at least 50 wt.%, more preferably at least 80 wt.% of a cationic copolymer based on dimethylaminoethyl methacrylate, more preferably a copolymer based on dimethylaminoethyl methacrylate, butyl methacrylate and methyl methacrylate (e.g. Eudragit^{®} E).

The dosage unit preferably comprises 3-30 wt.%, more preferably 4-15 wt.% of a saliva-resistant coating.

The performance of the dosage unit of the present invention can be determined in a dissolution test using simulated gastric fluid. This test is conducted using an USP2 apparatus and 900 mL simulated gastric fluid (pH 1.2). A detailed description of the test can be found in The United States Pharmacopeial Convention. General Chapter: 711 Dissolution: Apparatus 2. The United States Pharmacopeia and The National Formulary. USP 34-NF 29. Rockville, MD: The United States Pharmacopeial Convention, 2011. Preferably, in this dissolution test, the dosage unit of the present invention has released 15-60% of the local anaesthetic after 10 minutes and 60-100% of the local anaesthetic after 60 minutes. More preferably, in this dissolution test, the dosage unit of the present invention has released 20-50% of the local anaesthetic after 10 minutes and 80-100% of the local anaesthetic after 60 minutes.

The claims relate to a dosage unit for use in the treatment or prevention of malnutrition in subjects having adequate access to nutrition, or in the treatment or prevention of early or excessive satiety, said use comprising oral administration of a gastro-retentive oral dosage unit as described herein before.

The subject preferably is a human subject. More preferably, the subject is a human subject suffering from anorexia of aging, cachexia, malnutrition, post operative ileus, anorexia nervosa or sarcopenia.

In a further preferred embodiment, the subject is a human patient who has undergone surgery, especially abdominal surgery. Oral administration of the gastro-retentive dosage unit to this type of patient offers the advantage that it reduces the risk of developing post-operative ileus.

The use of the dosage unit preferably comprises at least once daily administration of the dosage unit.

According to a particularly preferred embodiment, the oral dosage unit is administered up to 30 minutes, more preferably up to 15 minutes and most preferably up to 10 minutes before the start of the consumption of a meal.

The use of the dosage unit in accordance with the present invention preferably comprises oral administration of the dosage unit to provide a dose of 30-400 mg of local anaesthetic, more preferably a dose of 50-300 mg of local anaesthetic.

The use preferably comprises oral administration of the dosage unit to provide a dose of 30-400 mg lidocaine, more preferably a dose of 50-300 mg lidocaine.

Yet another aspect which is not claimed relates to a process of preparing a dosage unit as described herein before, said process comprising:
- preparing a tableting mixture by intimately mixing components (a), (b), (c) and (d): and
- compressing the tableting mixture into tablets.

In a preferred embodiment of the present process, the tableting mixture is passed through a screen having a mesh size of 200-1000 µm, more preferably a mesh size of 300-800 µm, before the compressing step.

The present process preferably comprises the additional step of applying a saliva-resistant coating.

The saliva-resistant coating may be applied by means of dry powder coating or by coating with a liquid coating mixture comprising a solvent and coating polymer. Preferably, the saliva-resistant coating is applied by applying the liquid coating mixture onto the compressed tablet and allowing the solvent to evaporate.

The invention is further illustrated by the following examples.

### EXAMPLES

### Example 1

Oral dosage units according to the invention were prepared on the basis of the recipes shown in Table 1.

**Table 1**

| | **wt.%** | | | | | | |
|---|---|---|---|---|---|---|---|
| | **1** | **2** | **3** | **4** | **5** | **6** | **7** |
| Lidocaine | 38.02 | 36.76 | 36.76 | 33.33 | 33.33 | 33.33 | 33.33 |
| Sodium bicarbonate | 5.70 | 5.51 | 5.51 | 10.00 | 10.00 | 10.00 | 10.00 |
| Swelling polymer | | | | | | | |
| • Methocel K100LV ¹ | 15.21 | 14.71 | 14.71 | 15.00 | 15.00 | | |
| • Methocel K4M ² | | | | | | 15.00 | |
| • Methocel K15M CR Premium ³ | | | | | | | 15.00 |
| Filler | | | | | | | |
| • Avicel pH102 ⁴ | 38.02 | | | 20.00 | 15.67 | 35.67 | 35.67 |
| • Lactose monohydrate | | 36.76 | 36.76 | 15.67 | 20.00 | | |
| Disintegrant | | | | | | | |
| • Sodium croscarmellose | 2.09 | 5.33 | | 5.00 | | 5.00 | |
| • Sodium starch glycolate | | | 5.33 | | 5.00 | | 5.00 |
| Magnesium stearate | 0.95 | 0.92 | 0.92 | 1.00 | 1.00 | 1.00 | 1.00 |
| | | | | | | | |
| Tablet weight (in mg) | 263 | 272 | 272 | 300 | 300 | 300 | 300 |

| | | | | | | | |
|---|---|---|---|---|---|---|---|
| ¹ Low MW HPMC, yields a viscosity of 100 mPa·s (100 cP) at 2% in water (ex DuPont) ² Medium MW HPMC, yields a viscosity of 4,000 mPa·s (4,000 cP) at 2% in water (ex DuPont) ³ High MW HPMC, yields a viscosity of 15,000 mPa·s (15,000 cP) at 2% in water (ex DuPont) ⁴ Microcrystalline cellulose, partially depolymerized alphacellulose (ex DuPont) | | | | | | | |

These tablets were prepared as follows: Lidocaine and all excipients were screened, weighed, and all (except magnesium stearate) were transferred into a mixing vessel. After 15 minutes of mixing, magnesium stearate was added and mixing was continued for an additional 5 minutes to obtain a tableting blend. The blend was compressed into round tablets.

### Example 2

The tablet strength of the oral dosage units of Example 1 was determined using a Pharma Test^{™} hardness tester. In addition, tests were conducted to determine the floating time and disintegration time of the oral dosage units. The time after which the tablet starts floating was determined empirically in a glass beaker containing 100 ml simulated gastric fluid (pH 1.2).

The disintegration test was performed using an automated disintegration testing system including discs (Parmatron DisiTest).

The results of the tests are shown in Table 2.

**Table 2**

| | **1** | **2** | **3** | **4** | **5** | **6** | **7** |
|---|---|---|---|---|---|---|---|
| Tablet strength (N) | 7 | 80 | 78 | 75 | 90 | 95 | 80 |
| Floating time (min.) | 1 | 20 | 20 | 1 | 2 | 1 | 1 |
| Disintegration time (min.) | 20 | - | - | 28 | 35 | 20 | 40 |

### Example 3

The dissolution characteristics of the tablets of Example 1 was determined using an USP2 apparatus filled with 900 mL simulated gastric fluid (pH 1.2). The results are shown in Figure 1.

### Example 4

Lidocaine tablets were prepared on the basis of the recipe that is shown in Table 3, using the procedure as in Example 1.

**Table 3**

| | **wt.%** |
|---|---|
| Tablet core: | |
| • Lidocaine | 33.3 |
| • Sodium bicarbonate | 10 |
| • Methocel K100LV | 15 |
| • Avicel pH102 | 32.7 |
| • Lactose | 5 |
| • Sodium croscarmellose | 3 |
| • Magnesium stearate | 1 |

Next, the tablets were coated with a saliva-resistant coating at elevated temperature in a rotating drum by spraying the tablets with a coating solution (an aqueous solution of Eudragit EPO). After drying the weight of the tablets had increased by approximately 10 wt.%.

The dissolution characteristics of the coated tablet are shown in Figure 2.

### Example 5

Within the gastro-intestinal tract, local circuits play a role in the regulation of food intake and transport. Mechanical extension of the stomach as a result of food intake activates stretch receptors (neurons) which in turn induces satiety signals. The pharmacology of stretch receptors can be analyzed in fundus strip preparations.

Fresh stomach tissue (0-1h after slaughter) was collected from routinely slaughtered domestic pigs. The fundus region of the stomach was removed and washed with cold saline (0.9% NaCl) solution. Tissue was submerged in clean cold saline and transported to the laboratory (transport time 30 min.).

The mucosa and submucosa were removed from the muscularis (muscle layer). The muscularis was cut along the longitudinal muscle fibres in strips of approximately 1 by 5 cm. The strips were incubated for 15 min in a 37°C saline solution.

Thereafter, fundus strips were placed vertically in a transparent container, kept at 37°C. Stretching was ensured by a 5 g weight at the bottom of the strip. Test solutions were added into the container. Acetylcholine (10⁻⁴M) exposure was used to induce a contraction of the fundus tissue. Pictures were taken at 0, 5, 10, 15, 30, 45 and 60 minutes after the start of the experiment with a digital camera. The length of the strips was measured by a blinded assessor (unknowing of the treatment) using Photoshop software (Adobe). Values were expressed as percentage of baseline (t = 0 min).

Table 4 shows the relative length of the fundus strips (percentage of baseline) after exposure to saline + acetylcholine (Control) and saline + acetylcholine in combination with lidocaine (70 mg/L).

**Table 4**

| | **Strip length (as percentage of baseline)** | |
|---|---|---|
| **Time (minutes)** | **Control** | **Lidocaine** |
| 0 | 100 | 100 |
| 5 | 98.9 | 105.7** |
| 10 | 97.4 | 103.6* |
| 15 | 95.8 | 101.8* |
| 30 | 93.7 | 99.5 |
| 45 | 93.8 | 98.7 |
| 60 | 90.3 | 98.0* |

| | | |
|---|---|---|
| ** significant increase (p<0.01) * significant increase (p<0.05) | | |

These results show that the contraction induced by acetylcholine was reversed by the exposure to lidocaine.

### Example 6

Example 5 was repeated, except that this time lidocaine was replaced by benzocaine (70 mg/L). the results of the experiment are summarized in Table 5.

**Table 5**

| | **Strip length (as percentage of baseline)** | |
|---|---|---|
| **Time (minutes)** | **Control** | **Benzocaine** |
| 0 | 100 | 100 |
| 5 | 98.9 | 102.7* |
| 10 | 97.4 | 102.3* |
| 15 | 95.7 | 102.4** |
| 30 | 93.7 | 101.5* |
| 45 | 93.8 | 101.3** |
| 60 | 90.3 | 101.3** |

| | | |
|---|---|---|
| ** significant increase (p<0.01) * significant increase (p<0.05) | | |

### Example 7

Example 5 was repeated, except that this time lidocaine was replaced by bupivacaine (70 mg/L). the results of the experiment are summarized in Table 6.

**Table 6**

| | **Strip length (as percentage of baseline)** | |
|---|---|---|
| **Time (minutes)** | **Control** | **Bupivacaine** |
| 0 | 100 | 100 |
| 5 | 98.9 | 106.4*** |
| 10 | 97.4 | 107.1*** |
| 15 | 95.8 | 107.0*** |
| 30 | 93.7 | 106.8*** |
| 45 | 93.8 | 104.9*** |
| 60 | 90.3 | 101.9*** |

| | | |
|---|---|---|
| *** significant increase (p<0.001) | | |

### Example 8

Example 5 was repeated, except that this time lidocaine was replaced by Oxethazaine (70 mg/L). the results of the experiment are summarized in Table 7.

**Table 7**

| | **Strip length (as percentage of baseline)** | |
|---|---|---|
| **Time (minutes)** | **Control** | **Oxethazaine** |
| 0 | 100 | 100 |
| 5 | 98.9 | 104.5 |
| 10 | 97.4 | 104.8* |
| 15 | 95.8 | 104.3* |
| 30 | 93.7 | 101.3 |
| 45 | 93.8 | 98.2 |
| 60 | 90.3 | 94.6 |

| | | |
|---|---|---|
| * significant increase (p<0.05) | | |

### Example 9

Example 5 was repeated, except that this time lidocaine was replaced by prilocaine (70 mg/L). the results of the experiment are summarized in Table 8.

**Table 8**

| | **Strip length (as percentage of baseline)** | |
|---|---|---|
| **Time (minutes)** | **Control** | **Prilocaine** |
| 0 | 100 | 100 |
| 5 | 98.9 | 105.1 * |
| 10 | 97.4 | 105.8** |
| 15 | 95.8 | 106.2** |
| 30 | 93.7 | 106.3** |
| 45 | 93.8 | 104.1 * |
| 60 | 90.3 | 100.2* |

| | | |
|---|---|---|
| ** significant increase (p<0.01) * significant increase (p<0.05) | | |

### Example 10

The acid sensitivity of lidocaine was determined by exposing lidocaine to acetic acid (pH 2.5) for 1, 5 and 15 minutes before carrying out experiments on fundus strips, as in Example 5.

The results of the experiment are summarized in Table 9.

**Table 9**

| **Time (minutes)** | **Control** | **Lidocaine** | | |
|---|---|---|---|---|
| | | **1 min. acid** | **5 min. acid** | **15 min. acid** |
| 0 | 100 | 100 | 100 | 100 |
| 5 | 109.3 | 114.9 | 108.9 | 106.3 |
| 10 | 109.1 | 115.8 | 108.3 | 105.9 |
| 15 | 108.2 | 116.7 | 108.0 | 105.2 |
| 30 | 105.7 | 116.2* | 108.0 | 102.4 |
| 45 | 103.7 | 115.0* | 106.8 | 97.6 |
| 60 | 101.1 | 113.6* | 104.9 | 93.8 |

| | | | | |
|---|---|---|---|---|
| * significant increase (p<0.05) | | | | |

The results show that lidocaine is inactivated at low pH. Only at the shortest exposure time (1 min) lidocaine remains active and inhibits the acetylcholine induced contraction.

### Example 11

Oral dosage units according to the invention were prepared on the basis of the recipes shown in Table 10.

**Table 10**

| | **1** | **2** | **3** | **4** |
|---|---|---|---|---|
| Lidocaine hydrochloride monohydrate | 38.53 | 38.53 | 38.53 | 38.53 |
| Sodium bicarbonate | 5.00 | 5.00 | 5.00 | 5.00 |
| Methocel K100LV ¹ | 38.47 | 38.97 | 39.47 | 38.07 |
| Avicel pH102 ² | 15.00 | 15.00 | 15.00 | 15.00 |
| Sodium croscarmellose | 1.00 | 0.50 | 0.00 | 1.40 |
| Colloidal anhydrous SiO₂ | 1.00 | 1.00 | 1.00 | 1.00 |
| Magnesium stearate | 1.00 | 1.00 | 1.00 | 1.00 |

| | | | | |
|---|---|---|---|---|
| ¹ Low MW HPMC, yields a viscosity of 100 cP at 2% in water (ex DuPont) ² Microcrystalline cellulose, partially depolymerized alphacellulose (ex DuPont) | | | | |

These tablets were prepared as follows: Lidocaine was sieved in a tumbling mixer over a 600 µm sieve. The other components, except for the magnesium stearate were sieved in a tumbling mixer over a 800 µm sieve. The aforementioned components were mixed in the tumbling mixer for 15 minutes. Next, magnesium stearate was sieved over a 500 µm sieve and added to the powder mixture, followed by another 15 minutes of mixing in the tumbling mixer. The mixture so obtained was compressed into tablets of 10 mm biconvex, with an average weight of 318 mg.

The tablet strength, the floating time and disintegration time of the oral dosage units were determined in the same way as in Example 2. The results are shown in Table 11.

**Table 11**

| | **1** | **2** | **3** | **4** |
|---|---|---|---|---|
| Tablet strength (N) | 105 | 107 | 111 | 114 |
| Floating time (min.) | <8 | 8:40 | 7:30 | 4:50 |
| Disintegration time (min.) | 17:30 | 21:58 | 30:38 | 26:11 |

## Claims

1. A gastro-retentive oral dosage unit for use in the treatment or prevention of malnutrition in subjects having adequate access to nutrition, or in the treatment or prevention of early or excessive satiety, said use comprising oral administration of a dosage unit having a weight of 150-1,000 mg and comprising 70-100 wt.% of a core unit and 0-30 wt.% of a coating, the core unit comprising:
(a) 15-50 wt.% local anaesthetic;
(b) 2-20 wt.% of effervescent;
(c) 8-30 wt.% of hydrophilic polymer;
(d) 5-75 wt.% of excipient.

2. The dosage unit for use in the treatment or prevention according to claim 1, wherein the components (a) to (d) are intimately mixed.

3. The dosage unit for use in the treatment or prevention according to claim 1 or 2, wherein the dosage unit contains 30-400 mg local anaesthetic.

4. The dosage unit for use in the treatment or prevention according to any one of the preceding claims, wherein the local anaesthetic is selected from lidocaine, benzocaine, bupivacaine, oxethazaine, prilocaine, butanilicaine, carticaine, cinchocaine, clibucaine, etidocaine, mepivacaine, ropivacaine, tolycaine, trimecaine, vadocaine, articaine, levobupivacaine, amylocaine, cocaine, propanocaine, clormecaine, cyclomethycaine, proxymetacaine, amethocaine, butacaine, butoxycaine, butyl aminobenzoate, chloroprocaine, dimethocaine, oxybuprocaine, piperocaine, parethoxycaine, procaine, propoxycaine, tricaine and combinations thereof.

5. The dosage unit for use in the treatment or prevention according to claim 4, wherein the local anaesthetic is selected from lidocaine, benzocaine, bupivacaine, oxethazaine, prilocaine and combinations thereof.

6. The dosage unit for use in the treatment or prevention according to claim 5, wherein the local anaesthetic is lidocaine.

7. The dosage unit for use in the treatment or prevention according to any one of the preceding claims, wherein the effervescent is selected from bicarbonate salts, carbonate salts and combinations thereof.

8. The dosage unit for use in the treatment or prevention according to any one of the preceding claims, wherein the hydrophilic polymer is selected from hydroxypropyl methylcellulose, hydroxypropyl cellulose, hydroxyethyl cellulose, ethylcellulose, methylcellulose, carboxymethyl cellulose, carboxymethyl starch, polyethylene oxide, carbomer, polyvinyl alcohol, polyvinylpyrrolidone, vinylpyrrolidone-vinyl acetate copolymer, methacrylic acid copolymers, polyacrylic acids, carrageenan, natural gum, guar gum, tragacanth, acacia gum, locust bean gum, xanthan gum and combinations thereof.

9. The dosage unit for use in the treatment or prevention according to claim 8, wherein the hydrophilic polymer is hydroxypropyl methylcellulose.

10. The dosage unit for use in the treatment or prevention according to any one of the preceding claims, wherein the excipient includes a filler selected from lactose, microcrystalline cellulose, mannitol, starch and combinations thereof.

11. The dosage unit for use in the treatment or prevention according to claim 10, wherein the core unit contains 20-70 wt.% of filler.

12. The dosage unit for use in the treatment or prevention according to any one of the preceding claims, wherein the core unit contains a disintegrant selected from croscarmellose, sodium starch glycolate, crosspovidone and combinations thereof.

13. The dosage unit for use in the treatment or prevention according to claim 12, wherein the core unit contains 0.5-15 wt.% of disintegrant.

14. The dosage unit for use in the treatment or prevention according to any one of the preceding claims, wherein the combination of components (a) to (d) constitutes at least 75 wt.% of the core unit.

15. The dosage unit for use in the treatment or prevention according to any one of the preceding claims, wherein the dosage unit comprises 3-30 wt.% of a saliva-resistant coating.

## Patentansprüche

1. Gastro-retentive orale Dosierungseinheit zur Verwendung bei der Behandlung oder Vorbeugung von Mangelernährung bei Personen mit ausreichendem Zugang zu Nahrung oder bei der Behandlung oder Vorbeugung von frühzeitigem oder übermäßigem Sättigungsgefühl, wobei diese Verwendung die orale Verabreichung einer Dosierungseinheit umfasst, die ein Gewicht von 150-1000 mg aufweist und 70-100 Gew.-% einer Kerneinheit und 0-30 Gew.-% einer Beschichtung umfasst, wobei die Kerneinheit umfasst:
(a) 15-50 Gew.-% Lokalanästhetikum;
(b) 2-20 Gew.-% an Brausemittel;
(c) 8-30 Gew.-% an hydrophilem Polymer;
(d) 5-75 Gew.-% an Hilfsstoff.

2. Dosierungseinheit zur Verwendung bei der Behandlung oder Vorbeugung nach Anspruch 1, wobei die Komponenten (a) bis (d) intensiv vermischt sind.

3. Dosierungseinheit zur Verwendung bei der Behandlung oder Vorbeugung nach Anspruch 1 oder 2, wobei die Dosierungseinheit 30-400 mg Lokalanästhetikum enthält.

4. Dosierungseinheit zur Verwendung bei der Behandlung oder Vorbeugung nach einem der vorstehenden Ansprüche, wobei das Lokalanästhetikum ausgewählt ist aus Lidocain, Benzocain, Bupivacain, Oxethazain, Prilocain, Butanilicain, Carticain, Cinchocain, Clibucain, Etidocain, Mepivacain, Ropivacain, Tolycain, Trimecain, Vadocain, Articain, Levobupivacain, Amylocain, Kokain, Propanocain, Clormecain, Cyclomethycain, Proxymetacain, Amethocain, Butacain, Butoxycain, Butylaminobenzoat, Chloroprocain, Dimethocain, Oxybuprocain, Piperocain, Parethoxycain, Procain, Propoxycain, Tricain und Kombinationen davon.

5. Dosierungseinheit zur Verwendung bei der Behandlung oder Vorbeugung nach Anspruch 4, wobei das Lokalanästhetikum ausgewählt ist aus Lidocain, Benzocain, Bupivacain, Oxethazain, Prilocain und Kombinationen davon.

6. Dosierungseinheit zur Verwendung bei der Behandlung oder Vorbeugung nach Anspruch 5, wobei das Lokalanästhetikum Lidocain ist.

7. Dosierungseinheit zur Verwendung bei der Behandlung oder Vorbeugung nach einem der vorstehenden Ansprüche, wobei das Brausemittel ausgewählt ist aus Bicarbonatsalzen, Carbonatsalzen und Kombinationen davon.

8. Dosierungseinheit zur Verwendung bei der Behandlung oder Vorbeugung nach einem der vorstehenden Ansprüche, wobei das hydrophile Polymer ausgewählt ist aus Hydroxypropylmethylcellulose, Hydroxypropylcellulose, Hydroxyethylcellulose, Ethylcellulose, Methylcellulose, Carboxymethylcellulose, Carboxymethylstärke, Polyethylenoxid, Carbomer, Polyvinylalkohol, Polyvinylpyrrolidon, Vinylpyrrolidon-Vinylacetat-Copolymer, Methacrylsäure-Copolymeren, Polyacrylsäuren, Carrageen, Naturkautschuk, Guarkernmehl, Traganth, Gummiarabikum, Johannisbrotkernmehl, Xanthan und Kombinationen davon.

9. Dosierungseinheit zur Verwendung bei der Behandlung oder Vorbeugung nach Anspruch 8, wobei das das hydrophile Polymer Hydroxypropylmethylcellulose ist.

10. Dosierungseinheit zur Verwendung bei der Behandlung oder Vorbeugung nach einem der vorstehenden Ansprüche, wobei der Hilfsstoff ein Füllstoff ausgewählt aus Lactose, mikrokristalliner Cellulose, Mannitol, Stärke und Kombinationen davon beinhaltet.

11. Dosierungseinheit zur Verwendung bei der Behandlung oder Vorbeugung nach Anspruch 10, wobei die Kerneinheit 20-70 Gew.-% an Füllstoff enthält.

12. Dosierungseinheit zur Verwendung bei der Behandlung oder Vorbeugung nach einem der vorstehenden Ansprüche, wobei die Kerneinheit ein Sprengmittel ausgewählt aus Croscarmellose, Natriumstärkeglycolat, Crosspovidon und Kombinationen davon enthält.

13. Dosierungseinheit zur Verwendung bei der Behandlung oder Vorbeugung nach Anspruch 12, wobei die Kerneinheit 0,5-15 Gew.-% an Sprengmittel enthält.

14. Dosierungseinheit zur Verwendung bei der Behandlung oder Vorbeugung nach einem der vorstehenden Ansprüche, wobei die Kombination der Komponenten (a) bis (d) mindestens 75 Gew.-% der Kerneinheit ausmacht.

15. Dosierungseinheit zur Verwendung bei der Behandlung oder Vorbeugung nach einem der vorstehenden Ansprüche, wobei die Dosierungseinheit mindestens 3-30 Gew.-% einer speichelbeständigen Beschichtung umfasst.

## Revendications

1. Unité posologique orale gastro-rétentive pour utilisation dans le traitement ou la prévention de la malnutrition chez des sujets ayant **un** accès adéquat à la nutrition, ou dans le traitement ou la prévention d'une satiété précoce ou excessive, ladite utilisation comprenant une administration orale d'une unité posologique ayant un poids de 150 à 1 000 mg et comprenant 70 à 100 % en poids d'une unité centrale et 0 à 30 % en poids d'un enrobage, l'unité centrale comprenant :
(a) 15 à 50 % en poids d'anesthésique local ;
(b) 2 à 20 % en poids d'effervescent ;
(c) 8 à 30 % en poids de polymère hydrophile ;
(d) 5 à 75 % en poids d'excipient.

2. Unité posologique pour utilisation dans le traitement ou la prévention selon la revendication 1, où les composants (a) à (d) sont intimement mélangés.

3. Unité posologique pour utilisation dans le traitement ou la prévention selon la revendication 1 ou 2, où l'unité posologique contient 30 à 400 mg d'anesthésique local.

4. Unité posologique pour utilisation dans le traitement ou la prévention selon l'une quelconque des revendications précédentes, où l'anesthésique local est choisi parmi la lidocaïne, la benzocaïne, la bupivacaïne, l'oxéthazaïne, la prilocaïne, la butanilicaïne, la carticaïne, la cinchocaïne, la clibucaïne, l'étidocaïne, la mépivacaïne, la ropivacaïne, la tolycaïne, la trimécaïne, la vadocaïne, l'articaïne, la lévobupivacaïne, l'amylocaïne, la cocaïne, la propanocaïne, la clormécaïne, la cyclométhylcaïne, la proxymétacaïne, l'améthocaïne, la butacaïne, la butoxycaïne, l'aminobenzoate de butyle, la chloroprocaïne, la diméthocaïne, l'oxybuprocaïne, la pipérocaïne, la paréthoxycaïne, la procaïne, la propoxycaïne, la tricaïne et les combinaisons de ceux-ci.

5. Unité posologique pour utilisation dans le traitement ou la prévention selon la revendication 4, où l'anesthésique local est choisi parmi la lidocaïne, la benzocaïne, la bupivacaïne, l'oxéthazaïne, la prilocaïne et les combinaisons de celles-ci.

6. Unité posologique pour utilisation dans le traitement ou la prévention selon la revendication 5, où l'anesthésique local est la lidocaïne.

7. Unité posologique pour utilisation dans le traitement ou la prévention selon l'une quelconque des revendications précédentes, où l'effervescent est choisi parmi des sels de bicarbonate, des sels de carbonate et des combinaisons de ceux-ci.

8. Unité posologique pour utilisation dans le traitement ou la prévention selon l'une quelconque des revendications précédentes, où le polymère hydrophile est choisi parmi l'hydroxypropylméthylcellulose, l'hydroxypropylcellulose, l'hydroxyéthylcellulose, l'éthylcellulose, la méthylcellulose, la carboxyméthylcellulose, le carboxyméthylamidon, l'oxyde de polyéthylène, le carbomère, l'alcool polyvinylique, le polyvinylpyrrolidone, le copolymère de vinylpyrrolidone-acétate de vinyle, les copolymères d'acide méthacrylique, les acides polyacryliques, le carraghénane, la gomme naturelle, la gomme de guar, la gomme adragante, la gomme d'acacia, la gomme de caroube, la gomme xanthane et les combinaisons de ceux-ci.

9. Unité posologique pour utilisation dans le traitement ou la prévention selon la revendication 8, où le polymère hydrophile est l'hydroxypropylméthylcellulose.

10. Unité posologique pour utilisation dans le traitement ou la prévention selon l'une quelconque des revendications précédentes, où l'excipient inclut une charge choisie parmi le lactose, la cellulose microcristalline, le mannitol, l'amidon et les combinaisons de ceux-ci.

11. Unité posologique pour utilisation dans le traitement ou la prévention selon la revendication 10, où l'unité centrale contient 20 à 70 % en poids de charge.

12. Unité posologique pour utilisation dans le traitement ou la prévention selon l'une quelconque des revendications précédentes, où l'unité centrale contient un désintégrant choisi parmi la croscarmellose, le glycolate d'amidon sodique, la crospovidone et les combinaisons de ceux-ci.

13. Unité posologique pour utilisation dans le traitement ou la prévention selon la revendication 12, où l'unité centrale contient 0,5 à 15 % en poids de désintégrant.

14. Unité posologique pour utilisation dans le traitement ou la prévention selon l'une quelconque des revendications précédentes, où la combinaison des composants (a) à (d) constitue au moins 75 % en poids de l'unité centrale.

15. Unité posologique pour utilisation dans le traitement ou la prévention selon l'une quelconque des revendications précédentes, où l'unité posologique comprend 3 à 30 % en poids d'un enrobage résistant à la salive.
